# EUROPEAN PATENT SPECIFICATION

(11) **EP 4 169 475 B1**
(45) Date of publication and mention of the grant of the patent: **29.07.2026**
(21) Application number: 20940769.1
(22) Date of filing: 25.11.2020
(51) Int. Cl.: A61C 8/00, A61B 17/16, A61C 1/14

(54) **SURGICAL INSTRUMENT SET FOR DENTAL IMPLANTATION**
CHIRURGISCHES INSTRUMENTENSET ZUR ZAHNIMPLANTATION
ENSEMBLE CHIRURGICAL D'INSTRUMENTS POUR IMPLANTS DENTAIRES

(30) Priority: 18.06.2020 AM 20200053 U
(43) Date of publication of application: 26.04.2023
(73) Proprietor: Pahlavyan, Patrik, Yerevan, 0022 (AM)
(72) Inventor: Pahlavyan, Patrik, Yerevan, 0022 (AM)
(74) Representative: Makeeva, Svetlana
(86) International application number: PCT/IB2020/061130
(87) International publication number: WO 2021/255513

(56) References cited:
- KR-B1- 101 135 113
- KR-B1- 101 516 950
- RU-C2- 2 567 596
- US-A1- 2012 129 126
- US-A1- 2015 037 756
- US-A1- 2016 022 388
- US-A1- 2018 085 072
- US-B2- 8 827 704

## Description

### Technical field

The invention relates to the field of medicine, in particular to tooth implantation, and can be used for implanting teeth with a 3D navigation template.

### Prior art

Surgical instrument sets for dental implantation by means of using the method of navigational implantation are widely known, for example:
https://alfa-gate.net/product/conical-surgical-kit/
https://www.cad-ray.com/members/images/stmanual.pdf
http://www.biotech-dental.com/wp-content/uploads/2019/04/BR_ATLAS-SURGERY-2019_EN.pdf
http://dentiumusa.com/products/kits-and-instrument/digital-guide-surgery-kit-full.htm.

All of the above known surgical instrument sets include a mucotome, an osteotome, a pilot drill bit, a final drill bit and an implant driver with an implant, with a total number of at least 25 or more drill bits of different lengths and diameters.

A known set of surgical instruments for use in a surgical procedure to insert an implant, particularly a dental implant, in the bone of a patient comprises at least one drill hub configured for connection between a surgical drill handpiece and a drill. The drill is driveable by the drill handpiece via the drill hub. The drill hub has an end surface having defined therein a socket configured for receiving the drill. The socket has a defined depth to provide a predetermined extent of the drill from the end surface (US2016022388A1, IPC: A61C1/08, publ. on 28 January 2016).

This set contains several adapters of different lengths for final drills, which serve solely to control bone drilling depth. The working part of the drill acts as a guide, and during drilling, there is direct friction against the walls of the guide bushings, which leads to rapid tool wear, as well as to debris entering the bone tissue cavity.

Another instrument set for navigational dental implantation is described in the patent application (US2018085072 A1, IPC: A61B6/03, publ. on 29 March 2018). Fig. 8 of this patent application shows an instrument set for navigational dental implantation, where number 165 (from this point onward, the numbering of components is indicated in accordance with Fig. 8 of the closest prior art) shows drill bits that have different sizes, in length (rows A, B, C, D and E) and in diametrical dimensions (for example, 2.0 mm, 2.75 mm, 3.0 mm, 3.25 mm, 3.85 mm, 4.25 mm). Each drill bit has a limiter that engages with the upper surface of the guide tubes 190 to control the depth of its insertion.

When the dental plan is finalised, the specific series of drill bits 165 is selected for successive use with the surgical guide 110.

For example, to place a specific dental implant, the dental plan may include the use of B-2.0 mm and B-3.25 mm drill bits. The dimensions of the osteotomy are determined by means of using the final drill bit (B-3.25 mm), the dimensions of which correspond to the dental implant being installed. The surgical set additionally includes mucotomes 202 to remove gingival tissue; moreover, it includes pilot drill bits 204 to create a guide hole and, possibly, countersinks to shape an osteotomy hole. A series of drill bits and osteotomes serve to create an osteotomy, that is, a flat surface of the bone.

All the known surgical instrument sets, including the closest prior art, have common disadvantages, namely:
- a large number of instruments (25 or more), which complicates a dentist's work, as the probability of an error when choosing an instrument increases, and this also leads to a complication of the set and, accordingly, to an increase in its prime cost;
- the working part of the drill bit, during drilling, causes friction directly affecting the walls of the guide sleeves, which leads to rapid wear of the instrument, as well as to the ingress of sawdust into the bone tissue cavity. To reduce friction and wear of the drill bit, the gap between the drill bit and the guide sleeve is made large, which, in turn, leads to deviations from the correct guidance of the instrument.

### Disclosure of invention

*The problem addressed by the invention* is that of simplifying the surgical instrument set for dental implantation, reducing its prime cost and increasing the ease of use, the speed of operations and the reliability and efficiency of work performed by a dentist.

*The essence of the invention* is a surgical instrument set for dental implantation, comprising a mucotome, an osteotome, a pilot drill bit, final drill bits and an implant driver, which, *according to the invention,* additionally comprises an adapter for removable final drill bits and length limiters for the working part of the final drill bits, configured as rings of different widths. The adapter has a shank part and a cylindrical housing, in the cavity of which a locking fastener is disposed for affixing the final drill bits in the form of a spring element for latching to the shank of the final drill bit. The inner diameter of the limiters is made on the basis of the tight grip of the cylindrical housing of the adapter by a limiter.

### Brief description of drawings

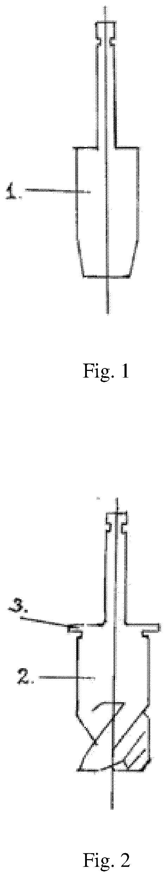

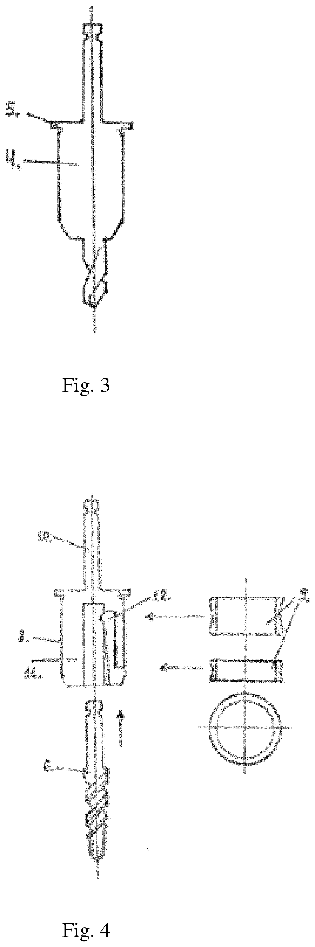

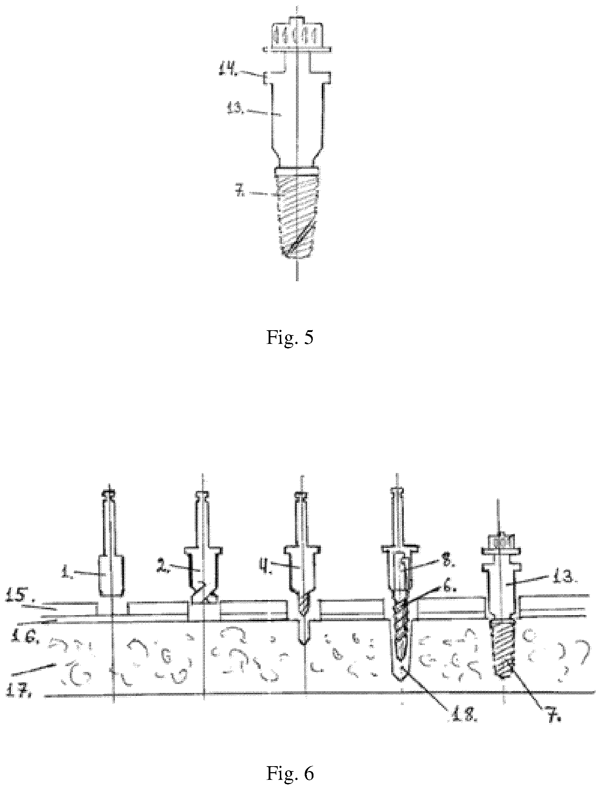

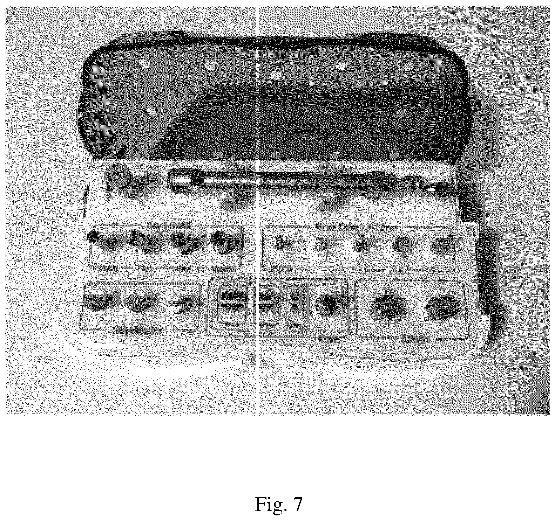

Fig. 1 shows the mucotome, Fig. 2 shows the osteotome, and Fig. 3 shows the pilot drill bit. Fig. 4 shows the final drill bit, the adapter and the limiters. Fig. 5 shows the implant driver with an implant, and Fig. 6 shows a protocol for performing implantation. Fig. 7 shows a photograph of the proposed instrument set.

### Carrying out the invention

One of the components of the instrument set is the mucotome (1), which is shown in Fig. 1 and the function of which is to cut the gingiva, thus creating a round hole. The mucotome (1) is a round cylindrical knife that cuts the gingiva while rotating. The optimum rotation speed is 200 rpm. This component is available in all surgical sets for dental implantation.

The next component of the instrument set is the osteotome (2), which is shown in Fig. 2 and the function of which is to align the bone ridge and to create a plateau for 0.5 mm above the level of the future implant, as the relief of the bone ridge is uneven. The osteotome (2) is a drill bit with a flat working part. The upper part of the drill bit has a limiting protrusion (3) to provide control of the height of the bone ridge alignment. The optimum rotation speed is 200 rpm. An osteotome is available in many surgical sets for dental implantation, including the closest prior art.

The next component of the instrument set is the pilot drill bit (4), which is shown in Fig. 3 and serves to create a guide hole. This is a short drill bit, with a diameter of 2.2 mm, that is placed around the guide sleeve of the surgical template, thereby ensuring the correct drilling direction. The upper part of the drill bit has a limiting protrusion (5) to provide control of the drilling depth. The optimum rotation speed is 300 rpm. A pilot drill bit is available in many surgical sets for dental implantation, including the closest prior art.

The next component of the instrument set is the final drill bit (6), which is shown in Fig. 4. The final drill bit serves to create a recess in the bone, having the size of the implant (7), both in length and in diameter. Final drill bits are available in all surgical sets for dental implantation, including the closest prior art, and a large number of them is available in known sets, which is due to the need to select drill bit parameters according to the implant length and diameter. For example, in the closest prior art we have chosen, the number of the final drill bits is 30, and, as already mentioned above when describing the closest prior art, the set of drill bits has different sizes, in length (rows A, B, C, D and E) and in diameter (2.0 mm, 2.75 mm, 3.0 mm, 3.25 mm, 3.85 mm, 4.25 mm).

Shown in the same Fig. 4, the following components of the instrument set: the adapter (8) for the removable final drill bits and the limiters (9) are the essential differences of the proposed invention. The adapter has a shank part (10) and a cylindrical housing (11), in the cavity of which a locking fastener (12) is disposed for affixing the final drill bits in the form of a spring element for latching to the shank of the final drill bit. The limiters (9) are configured as steel rings of different widths, wherein the inner diameter of the limiters is made on the basis of the tight grip of the cylindrical housing (11) of the adapter by the limiter (9). The optimum rotation speed is 350 rpm.

And, finally, the next component of the instrument set is the implant driver (13), which is shown in Fig. 5 (in Fig. 5, the implant driver is shown with an implant (7)). The implant driver consists of a guide part and an insertion limiting protrusion (14).

Fig. 6 shows a protocol for performing implantation with the use of the proposed surgical instrument set. First, the gingiva (15) is cut by means of using the mucotome (1) at a rotation speed of 200 rpm, thus creating a round hole. Further, the bone ridge (16) is aligned by means of using the osteotome (2), and a plateau is created for 0.5 mm above the level of the future implant. Then, with the pilot drill bit (4), which, in this embodiment example, is a short drill bit with a diameter of 2.2 mm, a small and narrow recess is made in the bone (17), thereby ensuring the correct direction for the insertion of the final drill bit.

The rotation speed of the pilot drill bit in this embodiment example is 300 rpm. Further, with the final drill bit (6), by means of using the adapter (8), a recess (18) is made in the bone (17), having the size of the implant (7), both in length and in diameter, while the depth of the recess is regulated by selecting the appropriate limiter (9). And, finally, the implant (7) is inserted into the recess (18) by means of using the implant driver (13).

The rationale with regard to the advantages of the proposed instrument set is given below. As mentioned above, the adapter (8) for the removable final drill bits and the limiters (9) are the essential differences of the proposed invention. The adapter has a shank part (10) and a cylindrical housing (11), in the cavity of which a locking fastener (12) is disposed for affixing the final drill bits, which can be successively changed during the operation. The locking fastener (12) is configured as a spring element for latching to the shank of the final drill bit.

The limiters (9) are configured as steel rings of different widths, which are put on the cylindrical housing (11) of the adapter. Depending on the selected limiter ring width, the length of the working part of the final drill bit is adjusted, and thus the depth of the recess (18) in the bone for placing the implant (7) is regulated.

As already mentioned above, in known sets, a large number of final drill bits is available, which is due to the need to select drill bit parameters according to both the implant length and its diameter.

Implant sizes vary from 6 mm to 14 mm in length and from 3 mm to 7 mm in diameter, depending on the implant manufacturer. Drill bit length: 6 mm, 7.5 mm, 8 mm, 10 mm, 11.5 mm, 13 mm, 14 mm, diameter: 2 mm, 2.4 mm, 2.8 mm, 3 mm, 3.2 mm, 3.4 mm, 3.6 mm, 3.8 mm, 4.2 mm, 4.8 mm etc. (depending on the manufacturer). Let us randomly take an implant manufacturer that produces implants with a length of 6 mm, 8 mm, 10 mm, and 12 mm and with a diameter of 3 mm, 3.6 mm, 4.2 mm, and 4.8 mm.

For fully-fledged drilling, it is necessary to have 16 drill bits with different sizes in the set (we multiply the number of drill bits with different lengths by the number of drill bits with different diameters). The availability of an adapter and limiters in the set, which are configured as steel rings of different widths and put on the adapter housing, solves the same problem by means of using 4 drill bits instead of 16.

Drilling depth is controlled by means of using limiters of different widths. In this embodiment example, two limiters configured as steel rings, with a width of 2 mm and with a width of 4 mm, have been used. For a drilling depth of 12 mm, no limiter is used, and only the drill bit with the appropriate diameter is selected. For a drilling depth of 10 mm, the limiter with a ring width of 2 mm is used, which is put on the adapter housing in advance (12 mm - 2 mm = 10 mm). To ensure a drilling depth of 8 mm, the limiter with a ring width of 4 mm is used (12 mm - 4 mm = 8 mm). To ensure a drilling depth of 6 mm, both limiters, with a ring width of 4 mm and with a ring width of 2 mm, are used (12 mm - 6 mm = 6 mm).

Therefore, the proposed invention has a number of advantages, namely:
1. the dentist initially sets the depth of drilling with the use of the limiters, which is very important for control during the operation so as not to damage anatomically important structures and tissues;
2. the surgical set is essentially simplified; in the given specific example, from 16 drill bits to 4. This, in turn, reduces the risk of a dentist's error when selecting drill bits and significantly reduces the time of the operation, as well as reduces the prime cost of the set;
3. due to the design feature of the adapter, the working part of the drill bit, during drilling, does not come into contact with the guide sleeve of the surgical template, which prevents wear of the instrument and the introduction of sawdust into the formed bone tissue bed.

At the same time, we note that the proposed simplified set is not inferior to other existing sets, which are equipped with a large number of instruments, and ensures fully-fledged high-quality implantation.

## Claims

1. A surgical instrument set for dental implantation, comprising a mucotome (1), an osteotome (2), a pilot drill bit (4), final drill bits (6) and an implant driver (13), wherein
the set additionally comprises an adapter (8) for removable final drill bits (6) and
limiters (9) for controlling the length of the working part of the final drill bits (6), configured as rings of different widths;
the adapter has a shank part (10) and a cylindrical housing (11), in the cavity of which a locking fastener (12) is disposed for affixing the final drill bits (6) in the form of a spring element for latching to the shank of the final drill bit (6),
wherein the limiters are configured to be put on the cylindrical housing (11) of the adapter (8),
wherein the inner diameter of the limiters (9) is made on the basis of the tight grip of the cylindrical housing (11) of the adapter (8) by a limiter (9).

## Patentansprüche

1. Ein chirurgisches Instrumentenset zur Zahnimplantation, welches ein Mukotom (1), ein Osteotom (2), einen Pilotbohrer (4), Endbohrer (6) und einen Implantathalter (13) umfasst, wobei
das Set zusätzlich einen Adapter (8) für abnehmbare Endbohrer (6) umfasst sowie
Begrenzer (9) zum Kontrollieren der Länge des Arbeitsbereichs der Endbohrer (6), die als Ringe unterschiedlicher Breite ausgeführt sind;
der Adapter weist einen Schaftteil (10) auf und ein zylindrisches Gehäuse (11), in dessen Hohlraum ein Verriegelungselement (12) zur Befestigung der Endbohrer (6) in Form eines Federelements angeordnet ist, das zum Einrasten am Schaft des Endbohrers (6) vorgesehen ist;
wobei die Begrenzer so konfiguriert sind, um am zylindrischen Gehäuse (11) des Adapters (8) angebracht zu werden;
wobei der Innendurchmesser der Begrenzer (9) so bestimmt wird, dass der Begrenzer (9) das zylindrischen Gehäuse (11) des Adapters (8) fest umgreift.

## Revendications

1. Ensemble chirurgical d'instruments pour implants dentaires, comprenant un mucotome (1), un ostéotome (2), un foret pilote (4), des forets finaux (6) et un porte-implant (13), dans lequel
l'ensemble comprend en outre un adaptateur (8) pour forets finaux amovibles (6) et
des butées (9) destinées à régler la longueur de la partie active des forets finaux (6), réalisées sous la forme de bagues de largeurs différentes ;
l'adaptateur comporte une partie de queue (10) et un corps cylindrique (11), dans la cavité duquel est disposé un organe de verrouillage (12) destiné à fixer les forets finaux (6), ledit organe se présentant sous la forme d'un élément ressort apte à s'encliqueter sur la queue du foret final (6),
dans lequel les butées sont configurées pour être montées sur le corps cylindrique (11) de l'adaptateur (8),
dans lequel le diamètre intérieur des butées (9) est dimensionné de manière à obtenir un ajustement serré d'une butée (9) sur le corps cylindrique (11) de l'adaptateur (8).
